# EUROPÄISCHE PATENTSCHRIFT

(11) **EP 3 419 598 B1**
(45) Veröffentlichungstag und Bekanntmachung des Hinweises auf die Patenterteilung: **06.11.2019**
(21) Anmeldenummer: 17710315.7
(22) Anmeldetag: 16.03.2017
(51) Int. Cl.: A61K 9/08, A61K 47/12, A61K 31/665

(54) **FOSFOMYCIN-FORMULIERUNG ZUR PARENTERALEN VERABREICHUNG**
FOSFOMYCIN FORMULATION FOR PARENTERAL ADMINISTRATION
FORMULE DE FOSFOMYCINE DESTINEE A L'ADMINISTRATION PARENTERALE

(30) Priorität: 16.03.2016 EP 16160698
(43) Veröffentlichungstag der Anmeldung: 02.01.2019
(60) Teilanmeldung: 19197649.7
(73) Patentinhaber: Georgopoulos, Apostolos, 1130 Wien (AT); Schifer, Albert, 1210 Wien (AT); Rous, Wolfgang, 1140 Wien (AT)
(72) Erfinder: Georgopoulos, Apostolos, 1130 Wien (AT); Schifer, Albert, 1210 Wien (AT); Rous, Wolfgang, 1140 Wien (AT)
(74) Vertreter: Sonn & Partner Patentanwälte
(86) Internationale Anmeldenummer: PCT/EP2017/056252
(87) Internationale Veröffentlichungsnummer: WO 2017/158099

(56) Entgegenhaltungen:
- WO-A2-2005/110022
- DE-U1-202008 012 514
- US-A1- 2015 057 242

## Beschreibung

Das Gebiet der vorliegenden Erfindung ist das der Fosfomycin-Formulierungen zur parenteralen Verabreichung, insbesondere zur intravenösen Verabreichung.

Das Antibiotikum Fosfomycin stellt eine strukturell völlig eigenständige Substanz innerhalb der Antibiotika dar und wurde ursprünglich aus *Streptomyces fradiae* isoliert. Parenteral wird Fosfomycin z.B. intravenös oder intramuskulär verabreicht und wird üblicherweise zur gezielten Therapie von Infektionen insbesondere im intensivmedizinischen Bereich verwendet, vor allem wenn Resistenzen oder Unverträglichkeiten in Bezug auf andere Antibiotika zu erwarten sind. Klinische Anwendungsgebiete für den Einsatz von Fosfomycin sind unter anderem: Im Bereich des zentralen Nervensystems Meningitis, Meningoencephalitis, Hirnabszess und Subduralempyem; in der Chirurgie postoperative Infektionen, Begleitinfektionen bei Tumoren sowie Prostatitis; in der Orthopädie und Traumatologie postoperative Knocheninfektionen (infizierte Osteosynthese, Endoprothese), Osteomyelitis, purulente Arthritis, Abszesse und Phlegmone; im Bereich des Respirationstraktes Bronchopneumonie, Lungenabszess und Nasennebenhöhlenentzündung; sowie Sepsis.

Fosfomycin-Dinatriumsalz ist ein gebräuchliches pharmazeutisch annehmbares Salz von Fosfomycin. Es ist ein weißes oder fast weißes, stark hygroskopisches Pulver, das in Wasser sehr gut löslich ist. In Wasser gelöstes Fosfomycin ist in Folge des hohen pH-Wertes der Lösung parenteral nicht verabreichbar. Um einen gewebeverträglichen bzw. venenverträglichen pH-Wert zu erreichen, wird daher bei Fosfomycinformulierungen zur parenteralen Verabreichung bei zur Zeit verfügbaren Präparaten Bernsteinsäure zugegeben.

Derzeit erfolgt der Zusatz von Bernsteinsäure als Pulver durch sterile Mischung mit Fosfomycin als Pulver. Diese Mischung wird als Pulver in Durchstechflaschen abgefüllt und in dieser Form in den Handel gebracht. In dieser Form als Trockensubstanz zur Infusionsbereitung zugelassen in einzelnen Ländern der Europäischen Union sind z.B. Fosfomycin Astro (Astro-Pharma GmbH, Wien, AT), Fosfomycin medicamentum (medicamenutum pharma GmbH, Allerheilgen, AT) und Fosfomycin Sandoz® (Sandoz GmbH, Kundl, AT) in Österreich, Fosfomycin Infectopharm® (Infectopharm Arzneimittel und Consilium GmbH, Heppenheim, DE) in Deutschland und Fomicyt® (Infectopharm Arzneimittel und Consilium GmbH, Heppenheim, DE) im Vereinigten Königreich.

Direkt vor der parenteralen Verabreichung müssen solche Trockensubstanzen von medizinischem Fachpersonal in einem geeigneten Lösungmittel aufgelöst werden.

Die derzeitige Vorgangsweise verursacht verschiedene Probleme. Die physikalische sterile Mischung von Trockensubstanzen ist sehr aufwändig, z.B. muss die Gleichmäßigkeit der Mischung gewährleistet werden, d.h. es darf nicht zur Entmischung oder zum Zusammenbacken des Pulvers kommen. Hierzu bedarf es einer komplizierten Technologie, die nur wenige Hersteller unter den erforderlichen Good Manufacturing Practice (GMP)-Bedingungen beherrschen. Dadurch kommt es laufend zu Lieferengpässen in der Versorgung mit Fosfomycin zur parenteralen Verabreichung. Beispielsweise sind seit Juni 2015 in Österreich laut Auskunft des Bundesamtes für Sicherheit im Gesundheitswesen alle der zur parenteralen Verabreichung zugelassenen Formulierungen von Fosfomycin nicht verfügbar, voraussichtlich bis zumindest März 2016 (Stand: Februar 2016).

Zusätzlich stellt der vom medizinischen Fachpersonal erforderte Weiterverarbeitungsschritt zur Infusionslösung ein Gesundheitsrisiko sowohl für das Fachpersonal als auch für den Patienten dar, insbesondere wenn die Fehleranfälligkeit durch hohe Arbeitsbelastung verstärkt ist: Zum ersten stellt der Weiterverarbeitungsschritt ein zusätzliches Risiko für Stichverletzungen beim Fachpersonal selbst dar. Zum zweiten ist das Risiko einer für den Patienten gefährlichen Kontamination oder Fehldosierung durch den zusätzlichen Weiterverarbeitungsschritt erhöht. Und zum dritten kann, wenn die Trockensubstanz wie üblich Fosfomycin-Dinatriumsalz enthält (übliche Dosen von ca. 4 bis 8 Gramm enthalten etwa 60 - 120 mmol Natriumionen), und die Trockensubstanz wegen eines Irrtums entgegen den Gebrauchsinformationen in physiologischer Kochsalzlösung aufgelöst und verabreicht wird, aufgrund des dadurch verursachten Natriumionenüberschusses eine mitunter lebensgefährliche Hypernatriämie beim Patienten verursacht werden.

Einige dieser Probleme werden auch durch die DE 20 2008 012 514 U1 erkannt. Jenes Dokument schlägt als Ansatz eine Verpackungseinheit zur Bereitstellung einer parenteral verabreichbaren, pharmakologisch akzeptablen pharmazeutischen Formulierung, die das Antibiotikum Fosfomycin und Bernsteinsäure oder eine andere biologisch kompatible Säure enthält, dadurch gekennzeichnet, dass die Einheit ein steril befülltes Behältnis mit in Pulverform vorliegendem Fosfomycin und ein steril befülltes Behältnis mit der in dem pharmakologisch akzeptablen Lösungsmittel gelösten Säure beinhaltet, vor.

Eine Aufgabe der vorliegenden Erfindung ist es nun, eine Fosfomycin-Formulierung zur parenteralen Verabreichung zur Verfügung zu stellen, welche die Nachteile des Standes der Technik wie u.a. Lieferengpässe durch aufwändige Herstellung und riskante Weiterverarbeitungsschritte direkt vor der Verabreichung, welche beispielsweise im Ansatz der DE 20 2008 012 514 U1 immer noch erforderlich sind, überwinden soll. D.h. es ist eine erfindungsgemäße Aufgabe, eine Fosfomycin-Formulierung zur parenteralen Verabreichung zur Verfügung zu stellen, die einfacher herzustellen und zu verabreichen ist.

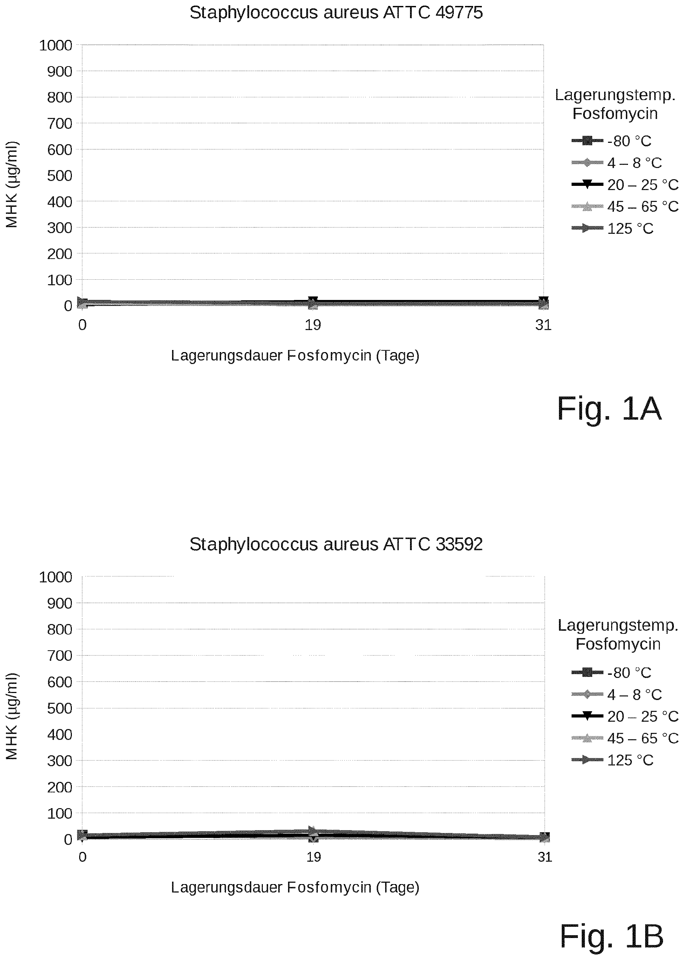

Es hat sich im Zuge der vorliegenden Erfindung überraschenderweise herausgestellt, dass Fosfomycin in wässriger Lösung viel stabiler ist als gemeinhin angenommen. Im Zuge der vorliegenden Erfindung wurde die Stabilität von Fosfomycin in wässriger Lösung in Anwesenheit einer Säure bei unterschiedlichen Temperaturen und Zeiträumen getestet. Sogar noch nach mehrfachem Autoklavieren bei 125°C und monatelanger Lagerung war Fosfomycin gegen die getesteten Bakterien wie *Staphylococcus* und *Escherichia* wirksam (vgl. Beispiel 2 und die Figuren 1A-1D).

Daher stellt die vorliegende Erfindung einen geschlossenen Behälter zur Verfügung, welcher eine wässrige Lösung gebrauchsfertig zur parenteralen Verabreichung beinhaltet, wobei in der Lösung zumindest ein pharmazeutisch annehmbares Salz von Fosfomycin und eine pharmazeutisch annehmbare Säure gelöst sind, wobei die Säure Bernsteinsäure ist, wobei die Lösung eine Fosfomycin-Gesamtdosis entsprechend 1 bis 15 Gramm Fosfomycin beinhaltet, wobei das Massenverhältnis von Bernsteinsäure zu Fosfomycin in der Lösung zwischen 30:1 und 50:1 liegt, wobei das Volumen der Lösung zwischen 1 ml und 100 ml / g Fosfomycin liegt. In der Lösung können weitere Wirkstoffe und/oder Hilfstoffe enthalten sein. Zweckmäßigerweise ist der Behälter hermetisch (bzw. "hermetisch versiegelt"), d.h. mit anderen Worten, dass kein Gasaustausch zwischen der Lösung und der Umgebung des Behälters möglich ist.

Um zur vorliegenden Erfindung zu gelangen, mussten die Erfinder ein im Stand der Technik stark verankertes Vorurteil überwinden:
Allen Fosfomycin-Formulierungen zur parenteralen Verabreichung im Stand der Technik ist gemein, dass deren Schöpfer davon ausgehen, dass Fosfomycin in Lösung so instabil ist, dass es in der Formulierung als Pulver vorliegen muss und erst kurz vor der Verabreichung aufgelöst werden darf. Dieses Vorurteil ist im Stand der Technik so stark verankert, dass beispielsweise unter enormem Aufwand die Trockenpulvermischung von Fosfomycin und Bernsteinsäure unter GMP-Bedingungen wie erläutert durchgeführt wird und Lieferengpässe wie oben dargelegt in Kauf genommen werden.

So empfiehlt der Public Assessment Report (PL 15011/0014 vom 1. September 2014) für Fomicyt® der Medicines & Healthcare products Regulatory Agency (MHRA) vor, nur frisch zubereitete Lösung zu verwenden. Die chemische Stabilität der gebrauchsfertigen, steril zubereiteten Fosfomycin-Lösung wird mit 12 Stunden bei 2-8°C unter Einhaltung des Lichtschutzes angegeben.

Die Gebrauchsinformation von Fosfomycin Astro, Fosfomycin Sandoz®, Fosfomycin medicamentum und Fosfomycin Infectopharm® empfehlen jeweils, nur frisch zubereitete Lösung zu verwenden. Die chemische Stabilität der gebrauchsfertigen, steril zubereiteten Lösung wird mit 24 Stunden bei 25°C unter Einhaltung von Lichtschutz angegeben.

Quentin et al. (Quentin, Claudine, et al. "Stability of fosfomycin and quinolones in peritoneal dialysis solution." Journal of Antimicrobial Chemotherapy 25.5 (1990): 878-880.) kommen zum Schluss, dass bestimmte Fosfomycin-Lösung 24 Stunden bei Raumtemperatur stabil sind.

Bailie et al. (Bailie, George R., and Michael P. Kane. "Stability of drug additives to peritoneal dialysate." Peritoneal dialysis international 15.8 (1995): 328-335.) geben ebenfalls dieses Ergebnis wieder.

Auch die folgenden Dokumente nehmen die vorliegende Erfindung nicht vorweg, weil sie keine konkrete Offenbarung mit Bezug zur vorliegenden Erfindung beinhalten, und führen nicht zu ihr hin, weil sie dem Fachmann keine konkreten Anhaltspunkte mit Bezug zur vorliegenden Erfindung liefern.

Die WO 2014/040947 A1 bezieht sich auf eine Aerosolerzeugungsvorrichtung mit Öffnungselement. In jenem Dokument wird Fosfomycin als einer unter Hunderten verschiedenartigster Wirkstoffe genannt, die gegebenenfalls mit der Aerosolerzeugungsvorrichtung verabreicht werden können. Beispielsweise wird die konkrete Kombination von geschlossenem Behälter, Wasser, pharmazeutisch annehmbaren Salz von Fosfomycin und pharmazeutisch annehmbarer Säure darin nicht offenbart.

Ähnliches gilt für die EP 2 062 608 A2, die eine Einwegampulle für eine Vorrichtung zur Erzeugung von Aerosolen offenbart.

Die WO 2008/071197 A1 bezieht sich auf Verfahren zur Behandlung von cystischer Fibrose oder bakterieller Lungenentzündung durch die pulmonäre Verabreichung von Fosfomycin, doch die konkrete Kombination von geschlossenem Behälter, Wasser, pharmazeutisch annehmbaren Salz von Fosfomycin und pharmazeutisch annehmbarer Säure wird darin nicht offenbart.

Die WO 2005/110022 A2 betrifft eine Fosfomycin-Aminglycosid-Kombination zur Behandlung von bakteriellen Atemwegsinfektionen. Die konkrete Kombination von geschlossenem Behälter, Wasser, pharmazeutisch annehmbaren Salz von Fosfomycin und pharmazeutisch annehmbarer Säure wird darin nicht offenbart. Die US 2015/0057242 A1 betrifft eine Fosfomycin-Aminglycosid-Kombination zur Behandlung von beatmungsassoziierter Pneumonie bzw. beatmungsassoziierter Bronchitis, die mittels Vernebler inhalativ verabreicht wird und dafür beispielsweise in Ampullen bereitgestellt wird. Allerdings offenbart das Dokument weder die Lagerbedingungen solcher Ampullen (z.B. in Bezug auf Lagertemperatur und -dauer) noch irgendwelche Versuchsergebnisse hinsichtlich der Lagerstabilität von Fosfomycin.

Die WO 2012/030513 A2 offenbart Verfahren zur Behandlung von bakteriellen Infektionen durch pulmonäre Verabreichung von Fusidinsäure, gegebenenfalls in Kombination mit Tobramycin, Amikacin, Fosfomycin oder Levofloxacin. Für Fosfomycin wird nur die orale Verabreichung und die pulmonäre Verabreichung vorgeschlagen (vgl. Abschnitt "Fosfomycin Dosage Forms", Absätze [0092] und [0093] jenes Dokuments), im Gegensatz zu anderen der genannten Wirkstoffe (vgl. Abschnitt "Amikacin Dosage Forms" unmittelbar davor und Abschnitt "Levofloxacin Dosage Forms" unmittelbar danach). Beispielsweise wird auch die konkrete Kombination von geschlossenem Behälter, Wasser, pharmazeutisch annehmbaren Salz von Fosfomycin und pharmazeutisch annehmbarer Säure darin nicht offenbart.

Die WO 2004/063036 A1 betrifft einen Sicherheitsbehälter für biologisch aktive Substanzen und Verfahren zu deren Herstellung. In jenem Dokument wird Fosfomycin als einer unter Tausenden verschiedenartigster Wirkstoffe genannt, die gegebenenfalls im Sicherheitsbehälter bereitgestellt werden können. Beispielsweise wird die konkrete Kombination von geschlossenem Behälter, Wasser, pharmazeutisch annehmbaren Salz von Fosfomycin und pharmazeutisch annehmbarer Säure darin nicht offenbart.

Zuletzt bezieht sich die WO 2010/048059 A1 auf Fosfomycin-Tobramycin-Kombinationen zur Behandlung oder Vorbeugung von ophthalmischen, otologischen und dermatologischen Infektionen. Die konkrete Kombination von geschlossenem Behälter, Wasser, pharmazeutisch annehmbaren Salz von Fosfomycin und pharmazeutisch annehmbarer Säure in derselben Lösung darin jedoch nicht offenbart. Bemerkenswerterweise wird in Bezug auf die Verpackung konkret offenbart, dass Fosfomycin für die Langzeitstabilisierung separat in einer Blisterpackung (d.h. trocken) aufbewahrt wird und erst vor der Verwendung zur Tobramycin-Lösung hinzugegeben wird (S. 24, 1. Absatz jenes Dokuments).

In einer bevorzugten Ausführungsform der vorliegenden Erfindung ist die wässrige Lösung, die der erfindungsgemäße geschlossene Behälter beinhaltet, zur Infusion oder Injektion in den Körper eines Säugetiers, insbesondere eines Menschen. Vorzugsweise ist dieses Säugetier bzw. dieser Mensch ein Patient, der an einer bakteriellen Infektion erkrankt ist.

In einer weiteren bevorzugten Ausführungsform der vorliegenden Erfindung ist die wässrige Lösung zu einer Verabreichung vorgesehen, die ausgewählt ist aus intravenöser Verabreichung, intramuskulärer Verabreichung, intraossärer Verabreichung, intravitrealer Verabreichung, intraperitonealer Verabreichung und intrathekaler Verabreichung. Davon ganz besonders bevorzugt ist die intravenöse Verabreichung.

Erfindungsgemäß liegt die wässrige Lösung gebrauchsfertig zur parenteralen Verabreichung vor. Das bedeutet insbesondere, dass keine Verdünnung, Vermischung (vor allem mit weiteren Stoffen), Auflösung, oder Rekonstitution vor der Verabreichung der Lösung mehr erforderlich ist. Gegebenenfalls ist jedoch, wenn die Lösung z.B. nicht in einer Fertigspritze oder in einem Infusionsbeutel vorliegt, beispielsweise ein Umfüllen in einen anderen, zur Verabreichung geeigneten Behälter wie z.B. eine Spritze erforderlich. Besonders bevorzugt liegt die wässrige Lösung gebrauchsfertig zur intravenösen oder gebrauchsfertig zur inhalativen Verabreichung vor.

Vorteilhafterweise ist der erfindungsgemäße geschlossene Behälter versiegelt, unter anderem aus Gründen der Manipulationssicherheit bzw. auch um versehentliches Wiederverwenden eines bereits geöffneten und dadurch möglicherweise kontaminierten Behälters zu verhindern. Der Ausdruck "versiegelt" bedeutet im Kontext der vorliegenden Erfindung, dass ein Öffnen des Behälters, das nach dem Versiegeln stattgefunden hat, nachweisbar, bevorzugt sogar mit freiem Auge sichtbar, ist, wobei die besagte Nachweisbarkeit bzw. Sichtbarkeit zumindest nicht ohne Aufwand bzw. ohne erheblichen Aufwand wieder rückgängig gemacht werden kann. Dem Fachmann sind Mittel zur Versiegelung im Arzneimittelbereich bekannt, darunter fallen beispielsweise Abziehfolien, Faserrissetiketten, Void-Siegel, Siegel mit Reißverschlussperforation und Perforation an Drehverschlüssen ("Frischesiegel"). Vorzugsweise erfüllt die Versiegelung des erfindungsgemäßen geschlossenen Behälters bzw. der erfindungsgemäße Behälter die Norm DIN EN 16679:2015-03.

Unter anderem aus ähnlichen Gründen wie im vorigen Absatz genannt ist in einer weiteren bevorzugten Ausführungsform der vorliegenden Erfindung der Behälter in einer Weise geschlossen, dass ein Öffnen des Behälters im Wesentlichen unwiderruflich ist (d.h. nur mit erheblichem Aufwand wieder rückgängig zu machen ist). Ein typisches Beispiel hierfür sind Brechampullen z.B. aus Plastik oder Glas. Eine Brechampulle ist beispielsweise aus der EP 0 243 580 A1 oder der EP 0 350 772 A1 bekannt.

In einer weiteren bevorzugten Ausführungsform ist der erfindungsgemäße geschlossene Behälter ausgewählt aus:
- einer Ampulle insbesondere aus Kunststoff oder Glas, vorzugsweise eine Brechampulle insbesondere aus Kunststoff oder Glas (jeder Behälter, der einen Verschluss aufweist, welcher aufgebrochen werden muss, fällt im Kontext der vorliegenden Erfindung unter den Begriff der "Brechampulle")
- einer Durchstechflasche (auch als "Vial" bezeichnet; vgl. z.B. die WO 2006/072440 A1), bevorzugt versiegelt mit einer Schutzkappe,
- einem Infusionsbeutel oder eine Influsionsflasche (z.B. mit Luer-Lock oder Gummistopfen, gegebenenfalls mit Graduierung) und
- einer Spritze, insbesondere einer Fertigspritze. Der Behälter kann aus einem transparenten oder intransparenten Material gefertigt sein. Vorzugsweise stellt der Behälter einen Lichtschutz für die Lösung dar (insbesondere im UV/VIS-Bereich). Der Behälter kann im Wesentlichen lichtundurchlässig bzw. lichtundurchlässig sein (insbesondere im UV/VIS-Bereich). Bevorzugt ist der Behälter ein Einwegbehälter bzw. zum einmaligen Gebrauch.

Zweckmäßigerweise weist der Behälter, insbesondere wenn er eine Durchstechflasche oder ein Infusionsbeutel ist, ein unversehrtes Septum beispielsweise aus Gummi auf, bzw. sind alle Septa, die der Behälter aufweist, unversehrt.

In einer weiteren bevorzugten Ausführungsform der vorliegenden Erfindung ist das pharmazeutisch annehmbare Salz von Fosfomycin ausgewählt aus Fosfomycin-Dinatriumsalz, Fosfomycin-Mononatriumsalz, einem Fosfomycin-Kaliumsalz, einem Fosfomycin-Lithiumsalz, Fosfomycin-Magnesiumsalz und Fosfomycin-Calciumsalz. Besonders bevorzugt sind die Natriumsalze, insbesondere Fosfomycin-Dinatriumsalz.

Hiermit wird als pharmazeutisch annehmbare Säure eine schwache Säure oder eine organische Säure, vorzugsweise eine schwache organische Säure beschrieben. Schwache Säuren sind insbesondere wegen ihrer Pufferwirkung bevorzugt. Insbesondere hat die, bevorzugt organische, Säure bei 25°C einen pKₛ-Wert von 2 bis 9, bevorzugt von 2,5 bis 8, mehr bevorzugt von 3 bis 7, noch mehr bevorzugt von 3,5 bis 6,5, insbesondere von 4 bis 6, vorzugsweise sind alle pKₛ-Werte der Säure (sofern die Säure mehrere pKₛ-Werte aufweist) unabhängig voneinander in einem dieser Intervalle. Zweckmäßigerweise ist die pharmazeutisch annehmbare Säure venenverträglich.

Erfindungsgemäß ist die pharmazeutisch annehmbare Säure Bernsteinsäure.

Zweckmäßigerweise sind in der wässrigen Lösung, die der erfindungsgemäße geschlossene Behälter beinhaltet, im Wesentlichen nur gelöst: ein pharmazeutisch annehmbares Salz von Fosfomycin und Bernsteinsäure gegebenenfalls mit Glucose oder Fructose (z.B. in einer Konzentration von 1% bis 5% w/v).

In einer weiteren bevorzugten Ausführungsform enthält die wässrige Lösung als Antibiotikum nur Fosfomycin. Vorzugsweise enthält die wässrige Lösung überhaupt nur Fosfomycin als Wirkstoff (mit der pharmazeutisch annehmbaren Säure als Hilfstoff), gegebenfalls mit weiteren Hilfsstoffen, insbesondere Glucose oder Fructose (z.B. in einer Konzentration von 1% bis 5% w/v).

In einer höchst bevorzugten Ausführungsform der vorliegenden Erfindung sind in der wässrigen Lösung im Wesentlichen nur Fosfomycin-Dinatriumsalz und Bernsteinsäure gelöst, gegebenenfalls mit Glucose oder Fructose (z.B. in einer Konzentration von 1% bis 5% w/v).

In einer weiteren bevorzugten Ausführungsform weist die wässrige Lösung (bevorzugt bei 25°C) einen pH-Wert von weniger als 10,0, bevorzugt von weniger als 9,0, mehr bevorzugt von weniger als 8,5, noch mehr bevorzugt von weniger als 8,0, insbesondere von weniger als 7,75 oder gar von höchstens 7,5 auf.

In einer weiteren bevorzugten Ausführungsform weist die wässrige Lösung (bevorzugt bei 25°C) einen pH-Wert von 3 bis 9, bevorzugt von 4 bis 8,5, mehr bevorzugt von 5 bis 8,5, insbesondere von 6 bis weniger als 8,0, auf.

In einer weiteren, besonders bevorzugten Ausführungsform weist die wässrige Lösung (bevorzugt bei 25°C) einen pH-Wert von 6,4 bis 8,4, bevorzugt von mehr als 6,5 und weniger als 8,0, eher bevorzugt von 6,9 bis 7,9, mehr bevorzugt von 7,0 bis 7,8, noch mehr bevorzugt von 7,1 bis 7,7, insbesondere von 7,2 bis 7,6 oder gar von 7,3 bis 7,5, auf. Insbesondere diese Ausführungsform ist für Infusion oder Injektion bzw. die intravenöse Verabreichung geeignet, wobei pH-Werte, die möglichst nahe beim normalen pH-Wert des menschlichen Blutes (7,35-7,45) liegen, besonders zweckmäßig sind.

In einer weiteren, bevorzugten Ausführungsform hat die wässrige Lösung (insbesondere wenn sie gebrauchsfertig zur intravenösen Verabreichung vorliegt) eine Osmolarität von höchstens 800 mosmol/L, bevorzugt höchstens 600 mosmol/L, mehr bevorzugt höchstens 500 mosmol/L, noch mehr bevorzugt höchstens 450 mosmol/L, insbesondere höchstens 400 mosmol/L oder gar höchstens 350 mosmol/L; zweckmäßigerweise bei einer gewissen Mindestosmolarität von z.B. mindestens 50 mosmol/L, mindestens 100 mosmol/L oder mindestens 200 mosmol/L. Ganz besonders bevorzugt ist die Lösung isotonisch oder hypotonisch (im Bezug auf menschliches Blutplasma), zweckmäßigerweise bei einer gewissen Mindestosmolarität von z.B. mindestens 50 mosmol/L, mindestens 100 mosmol/L oder mindestens 200 mosmol/L.

Die wässrige Lösung im erfindungsgemäßen geschlossenen Behälter enthält eine Fosfomycin-Gesamtdosis entsprechend 1 bis 15 Gramm Fosfomycin, insbesondere 1 bis 4 Gramm Fosfomycin. Insbesondere ist die Lösung als Einzeldosis vorgesehen. Das Volumen der Lösung liegt zwischen 1 ml und 100 ml / g Fosfomycin, bevorzugt zwischen 5 ml und 50 ml / g Fosfomycin, mehr bevorzugt zwischen 10 ml und 40 ml / g Fosfomycin, noch mehr bevorzugt zwischen 22,5 ml und 27,5 ml / g Fosfomycin, insbesondere bei im Wesentlichen 25 ml / g Fosfomycin.

In einer weiteren bevorzugten Ausführungsform ist die pharmazeutisch annehmbare Säure Bernsteinsäure und beträgt das Massenverhältnis von Bernsteinsäure zu Fosfomycin in der Lösung im Wesentlichen 40:1.

Zweckmäßigerweise ist der erfindungsgemäße geschlossene Behälter steril mit der Lösung befüllt worden. Insbesondere entspricht der geschlossene Behälter den am 15. Februar 2016 gültigen "EU Guidelines for Good Manufacturing Practice for Medicinal Products for Human and Veterinary Use", insbesondere Annex 1 in der Fassung "25 November 2008 (rev.)".

In einer weiteren bevorzugten Ausführungsform ist der Behälter gemäß Annex 1 der "EU Guidelines for Good Manufacturing Practice for Medicinal Products for Human and Veterinary Use" in der Fassung "25 November 2008 (rev.)" mit der wässrigen Lösung befüllt und geschlossen worden.

Durch die überraschende Erkenntnis, dass Fosfomycin in Lösung so stabil ist, ist es nun möglich, dieses in der industriellen Herstellung mit einem Antimykotikum in Lösung bereitzustellen (insbesondere gebrauchsfertig zur parenteralen bzw. inhalativen Verabreichung). Ein solches Kombinationsprodukt ist besonders gut geeignet für Patienten mit cystischer Fibrose (Mukoviszidose), deren Atemwege bzw. Lungen anfällig für Infektionen durch Bakterien und Pilze sind. Es ist daher bevorzugt, wenn die Lösung im geschlossenen Behälter ferner ein Antimykotikum enthält, welches vorzugsweise ausgewählt ist aus der Gruppe bestehend aus Terbinafin, Naftifin, Butenafin und pharmazeutisch annehmbaren Salzen davon. Die Lösung kann eine Gesamtdosis von 10 mg bis 5000 mg, bevorzugt von 20 mg bis 2000 mg, mehr bevorzugt von 30 mg bis 1000 mg, noch mehr bevorzugt von 40 mg bis 500 mg, insbesondere von 50 mg bis 250 mg des Antimykotikums beinhalten.

In einer besonders bevorzugten Ausführungsform ist das Antimykotikum Terbinafin oder ein pharmazeutisch annehmbares Salz davon. Es ist dabei bevorzugt, wenn die Lösung eine Terbinafin-Gesamtdosis entsprechend 10 mg bis 2000 mg Terbinafin, bevorzugt 20 mg bis 1000 mg Terbinafin, mehr bevorzugt 30 mg bis 500 mg Terbinafin, noch mehr bevorzugt 40 mg bis 250 mg Terbinafin, insbesondere 50 mg bis 200 mg oder gar 50 mg bis 100 mg Terbinafin beinhaltet. Das Massenverhältnis von Terbinafin zu Fosfomycin in der Lösung liegt üblicherweise zwischen 1:1 und 1:200, bevorzugt zwischen 1:2 und 1:100, mehr bevorzugt zwischen 1:3 und 1:75, noch mehr bevorzugt zwischen 1:4 und 1:50, insbesondere zwischen 1:5 und 1:25.

In einem weiteren Aspekt betrifft die vorliegende Erfindung eine Verpackung, umfassend mindestens einen erfindungsgemäßen geschlossenen Behälter. Die Verpackung kann unter anderem aus Karton, insbesondere bedrucktem Karton, gefertigt sein. Die Verpackung umfasst vorzugsweise eine Gebrauchsinformation. Die Verpackung stellt einen Lichtschutz für die Lösung bzw. den Behälter dar (insbesondere im UV/VIS-Bereich) und/oder der Behälter stellt einen Lichtschutz für die Lösung dar. Die Verpackung kann im Wesentlichen lichtundurchlässig bzw. lichtundurchlässig sein (insbesondere im UV/VIS-Bereich). Bevorzugt wahrt die Verpackung zusätzlich die Sterilität des geschlossenen Behälters (also auch seiner äußeren Oberfläche), z.B. wenn die Verpackung oder ein Teil davon als Plastikfolie ausgeführt ist.

Vorteilhafterweise ist die erfindungsgemäße Verpackung versiegelt, unter anderem aus Gründen der Manipulationssicherheit bzw. auch um versehentliches Wiederverwenden zu verhindern. Der Ausdruck "versiegelt" bedeutet in diesem Kontext, dass ein Öffnen der Verpackung, das nach dem Versiegeln stattgefunden hat, nachweisbar, bevorzugt sogar mit freiem Auge sichtbar, ist, wobei die besagte Nachweisbarkeit bzw. Sichtbarkeit zumindest nicht ohne Aufwand bzw. ohne erheblichen Aufwand wieder rückgängig gemacht werden kann. Dem Fachmann sind Mittel zur Versiegelung von Verpackungen im Arzneimittelbereich bekannt, darunter fallen beispielsweise Abziehfolien, Faserrissetiketten und Void-Siegel. Vorzugsweise erfüllt die Versiegelung der erfindungsgemäßen Verpackung bzw. die erfindungsgemäße Verpackung die Norm DIN EN 16679:2015-03.

In einer besonders bevorzugten Ausführungsform umfasst die Verpackung ferner ein Antimykotikum, insbesondere Terbinafin. Typischerweise ist das Antimykotikum in der Lösung mit Fosfomycin enthalten.

In einem weiterem Aspekt betrifft die vorliegende Erfindung das Lagern des erfindungsgemäßen geschlossenen Behälters bzw. der erfindungsgemäßen Verpackung bei einer Temperatur zwischen 0°C und 60°C, insbesondere bei einer Temperatur von 15°C bis 35°C oder bei Raumtemperatur, für zumindest eine Woche, bevorzugt für zumindest einen Monat, mehr bevorzugt für zumindest drei Monate. Die Lagerung findet unter Lichtschutz (insbesondere im UV-VIS-Bereich) statt.

In einem weiteren Aspekt betrifft die vorliegende Erfindung ein Verfahren, umfassend das sterile Befüllen des Behälters mit der Lösung und das Schließen des Behälters. Bevorzugt ist als weiterer Schritt das Versiegeln des geschlossenen Behälters, insbesondere entsprechend der Norm DIN EN 16679:2015-03, vorgesehen.

Zweckmäßigerweise wird die wässrige Lösung nicht aus einem steril entsprechend den am 15. Februar 2016 gültigen "EU Guidelines for Good Manufacturing Practice for Medicinal Products for Human and Veterinary Use", insbesondere entsprechend Annex 1 in der Fassung "25 November 2008 (rev.)", vermischten Pulvergemisch des pharmazeutisch annehmbaren Fosfomycin-Salzes und der pharmazeutisch annehmbaren Säure hergestellt.

In einem weiterem Aspekt betrifft die vorliegende Erfindung ein Verfahren zur Herstellung der erfindungsgemäßen Verpackung, umfassend das Durchführen des erfindungsgemäßen Verfahrens zur Herstellung des erfindungsgemäßen geschlossenen Behälters und das Verpacken des geschlossenen Behälters in die Verpackung. Bevorzugt ist als weiterer Schritt das Versiegeln der Verpackung, insbesondere entsprechend der Norm DIN EN 16679:2015-03, vorgesehen.

In einem weiteren Aspekt betrifft die vorliegende Offenbarung Fosfomycin zur Anwendung in der Vorbeugung oder Behandlung einer Infektion, insbesondere einer Infektion der unteren Atemwege bzw. der Lunge, in einem Patienten, der cystische Fibrose hat. Solche Patienten sind besonders anfällig für Atemwegs- bzw. Lungeninfektionen durch Bakterien bzw. Pilze, die immer wieder auftreten können und häufig auch chronisch werden. Einen Überblick über diese Problematik liefern beispielsweise Lyczak et al. ("Lung infections associated with cystic fibrosis." Clinical Microbiology Reviews 15.2 (2002): 194-222.) bzw. Gibson et al. ("Pathophysiology and management of pulmonary infections in cystic fibrosis." American Journal of Respiratory and Critical Care Medicine 168.8 (2003): 918-951). Aufgrund des erhöhten Risikos in Bezug auf Resistenzen ist es besonders sinnvoll, auch vergleichsweise eher ungebräuchliche Antibiotika wie Fosfomycin (bzw. im systemischen Einsatz vergleichsweise eher ungebräuchliche Antimykotika wie Terbinafin) zum einfachen Gebrauch bereits in Lösung (oder sogar gebrauchsfertig zur parenteralen bzw. inhalativen Verabreichung) zur Verfügung zu haben, wie dies durch die vorliegende Erfindung erreicht wird. Zusätzlich wird dadurch der Einsatz geringerer Dosen bzw. der gezieltere Einsatz ermöglicht, was wiederum das Resistenzrisiko vermindert.

In diesem Aspekt wird Fosfomycin, bzw. ein pharmazeutisch annehmbares Salz davon, üblicherweise in einer Lösung parenteral, intravenösbzw. per Inhalation an den Patienten verabreicht. Gegebenenfalls umfasst die Lösung auch ein Antimykotikum.

In einer besonders bevorzugten Ausführungsform dieser Offenbarung wird Fosfomycin bzw. ein pharmazeutisch annehmbares Salz davon gemeinsam mit Terbinafin bzw. einem pharmazeutisch annehmbaren Salz davon an den Patienten verabreicht. Typischerweise ist zumindest eine der Verabreichungen parenteral bzw. inhalativ. Zweckmäßigerweise liegt Fosfomycin bzw. ein pharmazeutisch annehmbares Salz davon und Terbinafin bzw. ein pharmazeutisch annehmbares Salz davon zur (bevorzugt parenteralen, insbesondere inhalativen) Verabreichung in derselben wässrigen Lösung vor.

### Weitere Definitionen:

Hierin ist "parenteral" in seiner breitesten Bedeutung ("unter Umgehung des Darmes") auszulegen, d.h. es werden durch diesen Begriff alle Arten der Verabreichung außer der oralen oder der rektalen umfasst.

Die wässrige Lösung ist im Sinne der vorliegenden Erfindung bevorzugt flüssig. Insbesondere unterscheidet sie sich von der Viskosität her nicht wesentlich von Wasser derselben Temperatur. Bevorzugt liegt die Viskosität der Lösung bei 20°C und Normaldruck zwischen 0,5 cP und 25 cP, bevorzugt bei etwa 1-2 cP, insbesondere bei etwa 1 cP. Bevorzugt ist die wässrige Lösung keine Suspension, Emulsion, Creme oder Gel.

Vorzugsweise ist das Wasser in der wässrigen Lösung Wasser zu Injektionszwecken, insbesondere gemäß Ph. Eur. (8. Auflage).

Die vorliegende Erfindung betrifft insbesondere die nachfolgenden, bevorzugten Ausführungsformen:
Ausführungsform 1. Geschlossener Behälter, beinhaltend eine wässrige Lösung gebrauchsfertig zur parenteralen Verabreichung, wobei in der Lösung zumindest ein pharmazeutisch annehmbares Salz von Fosfomycin und eine pharmazeutisch annehmbare Säure gelöst sind, wobei die Säure Bernsteinsäure ist, wobei die Lösung eine Fosfomycin-Gesamtdosis entsprechend 1 bis 15 Gramm Fosfomycin beinhaltet, wobei das Massenverhältnis von Bernsteinsäure zu Fosfomycin in der Lösung zwischen 30:1 und 50:1 liegt, wobei das Volumen der Lösung zwischen 1 ml und 100 ml / g Fosfomycin liegt.
Ausführungsform 2. Geschlossener Behälter gemäß Ausführungsform 1, wobei die Lösung gebrauchsfertig zur intravenösen Verabreichung oder gebrauchsfertig zur inhalativen Verabreichung vorliegt.
Ausführungsform 3. Geschlossener Behälter gemäß Ausführungsform 1 oder 2, wobei der geschlossene Behälter versiegelt ist.
Ausführungsform 4. Geschlossener Behälter gemäß einer der Ausführungsformen 1 bis 3, wobei der Behälter in einer Weise geschlossen ist, dass ein Öffnen des Behälters im Wesentlichen unwiderruflich ist.
Ausführungsform 5. Geschlossener Behälter gemäß einer der Ausführungsformen 1 bis 4, wobei der Behälter ausgewählt ist aus:
   - einer Ampulle insbesondere aus Kunststoff oder Glas, vorzugsweise eine Brechampulle insbesondere aus Kunststoff oder Glas,
   - einer Durchstechflasche, bevorzugt versiegelt mit einer Schutzkappe,
   - einem Infusionsbeutel und
   - einer Spritze, insbesondere einer Fertigspritze.
Ausführungsform 6. Geschlossener Behälter gemäß einer der Ausführungsformen 1 bis 5, wobei der Behälter, der vorzugsweise eine Durchstechflasche oder ein Infusionsbeutel ist, ein unversehrtes Septum beispielsweise aus Gummi aufweist.
Ausführungsform 7. Geschlossener Behälter gemäß einer der Ausführungsformen 1 bis 6, wobei der Behälter einen Lichtschutz für die Lösung darstellt, insbesondere im Wesentlichen lichtundurchlässig ist.
Ausführungsform 8. Geschlossener Behälter gemäß einer der Ausführungsformen 1 bis 7, wobei das pharmazeutisch annehmbare Salz von Fosfomycin ausgewählt ist aus Fosfomycin-Dinatriumsalz, Fosfomycin-Mononatriumsalz, einem Fosfomycin-Kaliumsalz, einem Fosfomycin-Lithiumsalz, Fosfomycin-Magnesiumsalz, und Fosfomycin-Calciumsalz.
Ausführungsform 9. Geschlossener Behälter gemäß einer der Ausführungsformen 1 bis 8, wobei in der Lösung im Wesentlichen nur gelöst sind ein pharmazeutisch annehmbares Salz von Fosfomycin und eine pharmazeutisch annehmbare Säure, gegebenenfalls mit Glucose oder Fructose.
Ausführungsform 10. Geschlossener Behälter gemäß Ausführungsform 9, wobei in der Lösung im Wesentlichen nur Fosfomycin-Dinatriumsalz und Bernsteinsäure gelöst sind, gegebenenfalls mit Glucose oder Fructose.
Ausführungsform 11. Geschlossener Behälter gemäß einer der Ausführungsformen 1 bis 10, wobei die Lösung (bevorzugt bei 25°C) einen pH-Wert von 6,4 bis 8,4, bevorzugt von mehr als 6,5 und weniger als 8,0, eher bevorzugt von 6,9 bis 7,9, mehr bevorzugt von 7,0 bis 7,8, noch mehr bevorzugt von 7,1 bis 7,7, insbesondere von 7,2 bis 7,6 oder gar von 7,3 bis 7,5, aufweist; oder wobei die Lösung (bevorzugt bei 25°C) einen pH-Wert von weniger als 10,0, bevorzugt von weniger als 9,0, mehr bevorzugt von weniger als 8,5, noch mehr bevorzugt von weniger als 8,0, insbesondere von weniger als 7,75 oder gar von höchstens 7,5 aufweist; oder wobei die Lösung (bevorzugt bei 25°C) einen pH-Wert von 3 bis 9, bevorzugt von 4 bis 8,5, mehr bevorzugt von 5 bis 8,5, insbesondere von 6 bis weniger als 8,0, aufweist.
Ausführungsform 12. Geschlossener Behälter gemäß einer der Ausführungsformen 1 bis 11, wobei die Lösung eine Fosfomycin-Gesamtdosis entsprechend 1 bis 4 Gramm Fosfomycin beinhaltet.
Ausführungsform 13. Geschlossener Behälter gemäß einer der Ausführungsformen 1 bis 12, wobei die pharmazeutisch annehmbare Säure Bernsteinsäure ist und das Massenverhältnis von Bernsteinsäure zu Fosfomycin in der Lösung bei im Wesentlichen 40:1 liegt.
Ausführungsform 14. Geschlossener Behälter gemäß einer der Ausführungsformen 1 bis 13, wobei der Behälter steril mit der Lösung befüllt worden ist.
Ausführungsform 15. Geschlossener Behälter gemäß einer der Ausführungsformen 1 bis 14, wobei der geschlossene Behälter den am 15. Februar 2016 gültigen "EU Guidelines for Good Manufacturing Practice for Medicinal Products for Human and Veterinary Use", insbesondere Annex 1 in der Fassung "25 November 2008 (rev.)", entspricht.
Ausführungsform 16. Geschlossener Behälter gemäß einer der Ausführungsformen 1 bis 15, wobei der Behälter gemäß Annex 1 der "EU Guidelines for Good Manufacturing Practice for Medicinal Products for Human and Veterinary Use" in der Fassung "25 November 2008 (rev.)" mit der Lösung befüllt und geschlossen worden ist.
Ausführungsform 17. Geschlossener Behälter gemäß einer der Ausführungsformen 1 bis 8 und 11 bis 16, wobei in der Lösung ferner ein Antimykotikum gelöst ist.
Ausführungsform 18. Geschlossener Behälter gemäß Ausführungsform 17, wobei das Antimykotikum ausgewählt ist aus der Gruppe bestehend aus Terbinafin, Naftifin, Butenafin und pharmazeutisch annehmbaren Salzen davon.
Ausführungsform 19. Geschlossener Behälter gemäß Ausführungsform 18, wobei das Antimykotikum Terbinafin oder ein pharmazeutisch annehmbares Salz davon ist.
Ausführungsform 20. Geschlossener Behälter gemäß einer der Ausführungsformen 17 bis 19, wobei die Lösung eine Gesamtdosis von 10 mg bis 5000 mg, bevorzugt von 20 mg bis 2000 mg, mehr bevorzugt von 30 mg bis 1000 mg, noch mehr bevorzugt von 40 mg bis 500 mg, insbesondere von 50 mg bis 250 mg des Antimykotikums beinhaltet.
Ausführungsform 21. Geschlossener Behälter gemäß Ausführungsform 19, wobei die Lösung eine Terbinafin-Gesamtdosis entsprechend 10 mg bis 2000 mg Terbinafin, bevorzugt 20 mg bis 1000 mg Terbinafin, mehr bevorzugt 30 mg bis 500 mg Terbinafin, noch mehr bevorzugt 40 mg bis 250 mg Terbinafin, insbesondere 50 mg bis 200 mg oder gar 50 mg bis 100 mg Terbinafin beinhaltet.
Ausführungsform 22. Geschlossener Behälter gemäß Ausführungsform 19, wobei das Massenverhältnis von Terbinafin zu Fosfomycin in der Lösung zwischen 1:1 und 1:200, bevorzugt zwischen 1:2 und 1:100, mehr bevorzugt zwischen 1:3 und 1:75, noch mehr bevorzugt zwischen 1:4 und 1:50, insbesondere zwischen 1:5 und 1:25 liegt.
Ausführungsform 23. Verpackung, umfassend zumindest einen geschlossenen Behälter gemäß einer der Ausführungsformen 1 bis 16 und vorzugsweise eine Gebrauchsinformation; wobei die Verpackung einen Lichtschutz für die Lösung bzw. den geschlossenen Behälter darstellt, insbesondere im Wesentlichen lichtundurchlässig ist, und/oder wobei der Behälter einen Lichtschutz für die Lösung darstellt, insbesondere im Wesentlichen lichtundurchlässig ist.
Ausführungsform 24. Verpackung gemäß Ausführungsform 23, ferner umfassend ein Antimykotikum, insbesondere Terbinafin, vorzugsweise wobei das Antimykotikum wie in einer der Ausführungsformen 17 bis 22 definiert vorliegt.
Ausführungsform 25. Verpackung gemäß Ausführungsform 23 oder 24, wobei die Verpackung im Wesentlichen lichtundurchlässig ist.
Ausführungsform 26. Verfahren, umfassend das Lagern des geschlossenen Behälters einer der Ausführungsformen 1 bis 22 bzw. der Verpackung einer der Ausführungsformen 23 bis 25 bei einer Temperatur zwischen 0°C und 60°C, insbesondere bei einer Temperatur von 15°C bis 35°C oder bei Raumtemperatur, für zumindest eine Woche, bevorzugt für zumindest einen Monat, mehr bevorzugt für zumindest drei Monate, wobei die Lagerung unter Lichtschutz statt findet.
Ausführungsform 27. Verfahren zur Herstellung des geschlossenen Behälters eines der Ausführungsformen 1 bis 22, umfassend das sterile Befüllen des Behälters mit der Lösung und das Schließen des Behälters.
Ausführungsform 28. Verfahren zur Herstellung gemäß Ausführungsform 27, ferner umfassend das Versiegeln des geschlossenen Behälters.
Ausführungsform 29. Verfahren zur Herstellung gemäß einer der Ausführungsformen 27 bis 28, wobei die Lösung nicht aus einem steril entsprechend den am 15. Februar 2016 gültigen "EU Guidelines for Good Manufacturing Practice for Medicinal Products for Human and Veterinary Use", insbesondere entsprechend Annex 1 in der Fassung "25 November 2008 (rev.)", vermischten Pulvergemisch des pharmazeutisch annehmbaren Fosfomycin-Salzes und der pharmazeutisch annehmbaren Säure hergestellt wird. Ausführungsform 30. Verfahren zur Herstellung der Verpackung gemäß einer der Ausführungsformen 23 bis 25, umfassend das Durchführen des Verfahrens gemäß einer der Ausführungsformen 27 bis 29, wodurch besagter geschlossener Behälter erhalten wird, und das Verpacken des geschlossenen Behälters in die Verpackung.

Die vorliegende Offenbarung betrifft insbesondere die nachfolgenden Ausführungsformen.

Ausführungsform 31. Fosfomycin zur Anwendung in der Vorbeugung oder Behandlung einer Infektion, insbesondere einer Infektion der unteren Atemwege, in einem Patienten, der cystische Fibrose hat, wobei das Fosfomycin bzw. ein pharmazeutisch annehmbares Salz davon in einer Lösung, wie sie in einer der Ausführungsformen 1 bis 16 definiert ist, parenteral, insbesondere intravenös oder inhalativ, an den Patienten verabreicht wird. Ausführungsform 32. Fosfomycin zur Anwendung in der Vorbeugung oder Behandlung einer Infektion, insbesondere einer Infektion der unteren Atemwege, in einem Patienten, der cystische Fibrose hat, wobei das Fosfomycin bzw. ein pharmazeutisch annehmbares Salz davon gemeinsam mit einem Antimykotikum in einer Lösung, wie sie in einer der Ausführungsformen 17 bis 22 definiert ist, parenteral, insbesondere intravenös oder inhalativ, an den Patienten verabreicht wird.

Ausführungsform 33. Fosfomycin zur Anwendung in der Vorbeugung oder Behandlung einer Infektion, insbesondere einer Infektion der unteren Atemwege, in einem Patienten, der cystische Fibrose hat, wobei Fosfomycin bzw. ein pharmazeutisch annehmbares Salz davon gemeinsam mit Terbinafin bzw. einem pharmazeutisch annehmbaren Salz davon bevorzugt parenteral, insbesondere intravenös oder inhalativ, an den Patienten verabreicht werden. Ausführungsform 34. Fosfomycin zur Anwendung gemäß Ausführungsform 33, wobei Fosfomycin bzw. ein pharmazeutisch annehmbares Salz davon und Terbinafin bzw. ein pharmazeutisch annehmbares Salz davon zur Verabreichung in derselben wässrigen Lösung vorliegen.

Die Erfindung wird ferner durch die folgenden Beispiele und Figuren, auf welche sie selbstverständlich nicht eingeschränkt ist, veranschaulicht.

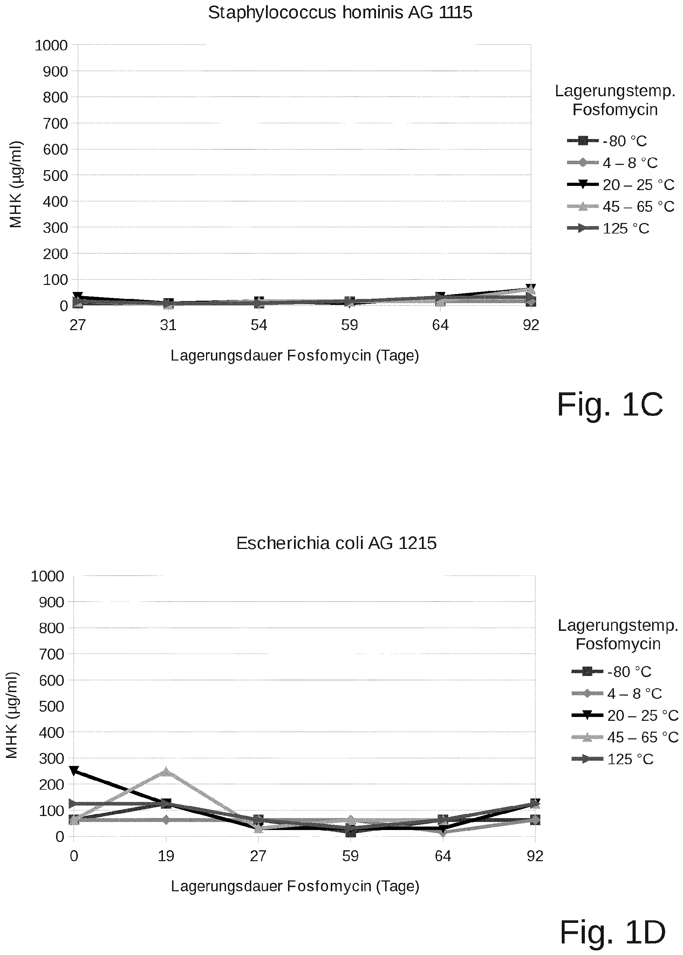

**Figuren 1A-1D****:** Minimale Hemmkonzentration (MHK) einer Fosfomycin-Säure-Lösung in Abhängigkeit von Lagerungsdauer und -temperatur in Bezug auf *Staphylococcus aureus* ATTC 49775 (Fig. 1A), *Staphylococcus aureus* ATTC 33592 (Fig. 1B), *Staphylococcus hominis* AG 1115 (Fig. 1C) und *Escherichia coli* AG 1215 (Fig. 1D). Je niedriger die MHK eines Antibiotikums, desto stärker ist bekanntermaßen die antimikrobielle Wirkung des Antibiotikums. Die im Zuge der vorliegenden Erfindung gemessenen MHK-Werte gegenüber den getesteten Bakterienstämmen lagen im Vergleich zum Anfangswert im üblichen Streuungsbereich der Versuchsanordnung, d.h. es konnte im beobachteten Zeitraum überraschenderweise keine durch die Lagerung verursachte Verschlechterung der MHK beobachtet werden. Folglich behält eine Lösung von Fosfomycin und einer Säure bei Raumtemperatur (und sogar bei Autoklavierung bzw. bei extremen Temperaturbedingungen) über einen Zeitraum von mindestens drei Monaten die antimikrobielle Wirksamkeit bei, was dem im Stand der Technik stark verankerten Vorurteil widerspricht, welches besagt, dass Fosfomycin instabil wäre.

### Beispiel 1 - Herstellung

In 500 L Wasser für Injektionszwecke gemäß Ph. Eur. (8. Auflage) werden 26,4 kg Fosfomycin-Dinatrium (entspricht 20 kg Fosfomycin) und 500 g Bernsteinsäure unter sterilen Bedingungen gelöst. Diese Lösung wird unter Sterilbedingungen in 5000 Infusionsflaschen (Einzelvolumen: 100 ml) abgefüllt. Die Infusionsflaschen werden mit je einem geeigneten Gummistopfen verschlossen und zu je 10 Stück in einen Karton verpackt.

Der pH-Wert dieser Lösung liegt bei 7,5. Die in den Infusionsflaschen beinhaltete Lösung ist gebrauchsfertig zur intravenösen Verabreichung und kann monatelang bei Raumtemperatur gelagert werden. Wenn fermentativ hergestelltes Fosfomycin verwendet wird, wird in der Endabfüllung unter Sterilbedingungen in die Infusionsflasche ein Sterilfilter vorgeschaltet.

### Beispiel 2 - Stabilitätsuntersuchungen

Zielsetzung der vorliegenden Untersuchungen war, ob die antimikrobielle Aktivität von Fosfomycin mit einem für die intravenöse Anwendung geeigneten pH-Wert in einem für die intravenöse Anwendung geeigneten Lösungsmittel erhalten bleibt. Dafür wurde Fosfomycin Dinatrium (14,5 mmol Natrium/g) und Bernsteinsäure zur pH-Anpassung (0,025 Gramm Bernsteinsäure pro Gramm Fosfomycin) verwendet. Der pH-Wert dieser Lösung lag bei 7,5.

Es wurde eine Stammlösung von 4g Fosfomycin Dinatrium und Bernsteinsäure (0,025 Gramm Bernsteinsäure pro Gramm Fosfomycin, entsprechend ca. 3g Fosfomycin) in 10 ml destilliertem Wasser hergestellt und in 5 Kryoröhrchen zu je 2 ml aufgeteilt und die Lösung wurde bei unterschiedlichen Temperaturen wie folgt exponiert:
Je 2 ml der Probe wurden bei -80° C, bei Kühlschranktemperatur (2-8° C), bei Raumtemperatur (20-25° C) und bei 45-65° C bis Versuchsende aufbewahrt. Weitere 2 ml der Stammlösung wurden bei Kühlschranktemperatur (2-8° C) gelagert und vor den Versuchen bei 125° C über 30 Minuten autoklaviert. Danach wurden alle Proben auf ihre antimikrobielle Wirksamkeit geprüft.

Zur Bestimmung der antimikrobiellen Wirksamkeit von Fosfomycin wurde die minimale Hemmkonzentration (MHK) bei den 4 genannten Bakterienstämmen mittels Röhrchenverdünnungsverfahren in Mikrotiterplatten unter Verwendung von Müller-Hinton-Bouillon bestimmt. Für die Beimpfung der Mikrotiterplatten wurde das Inokulum auf 1,5^{∗}104 Keime/ml eingestellt. Die Auswertung erfolgte nach 24 Stunden Inkubation bei 37° C.

Es wurden Proben, beginnend bei 2.000µg/ml Prüfsubstanz jeweils um den Faktor 2 verdünnt. Es wurden Verdünnungsreihen von 2.000 µg/ml bis 1,95 µg/ml hergestellt. Zum Nachweis der antimikrobiellen Aktivität und der Stabilität einer Lösung von Fosfomycin und Bernsteinsäure in einem für die intravenöse Verabreichung geeigneten Lösungsmittel wurden die MHK-Werte nach einer Inkubation über 24 Stunden bei 37° C ermittelt.

Folgende Bakterienstämme wurden für die Untersuchungen verwendet:
*Staphylococcus aureus* Nr. ATTC 49775,
*Staphylococcus aureus* Nr. ATTC 33592,
*Staphylococcus hominis* Nr. AG 1115 und
*Escherichia coli* Nr. AG 1215.

Die Ergebnisse sind in Tabelle 1 dargestellt, und werden auch in den Figuren 1A-1D veranschaulicht. Die gemessenen MHK-Werte gegenüber den getesteten Bakterien liegen im Vergleich zum Anfangswert im üblichen Streuungsbereich der Versuchsanordnung, d.h. es konnte im beobachteten Zeitraum überraschenderweise keine durch die Lagerung verursachte Verschlechterung der MHK beobachtet werden. Folglich behält eine Lösung von Fosfomycin und einer Säure bei Raumtemperatur (und sogar bei Autoklavierung bzw. bei extremen Temperaturbedingungen) über einen Zeitraum von mindestens drei Monaten die antimikrobielle Wirksamkeit bei, was dem im Stand der Technik stark verankerten Vorurteil widerspricht, welches besagt, dass Fosfomycin instabil wäre.

**Tabelle 1: In der Tabelle sind die MHK-Werte der getesteten Bakterienstämme abhängig von den Fosfomycin-Lagerungsbedingungen (Dauer und Temperatur) aufgeführt.**

| **Lagerungsabhängige minimale Hemmkonzentrationen (in µg/ml) von Fosfomycin** | | | | | | | | |
|---|---|---|---|---|---|---|---|---|
| **Lagertemp. (°C)** | **Lagerungsdauer von Fosfomycin in Tagen** | | | | | | | |
| | **0** | **19** | **27** | **31** | **54** | **59** | **64** | **92** |
| | | | | | | | | |

| ***Staphylococcus aureus* ATTC 49775** | | | | | | | | |
|---|---|---|---|---|---|---|---|---|
| **-80** | 7,8 | 3,9 | ----- | 3,9 | ----- | ----- | ----- | ----- |
| **4 - 8** | 7,8 | 3,9 | ----- | 3,9 | ----- | ----- | ----- | ----- |
| **20 - 25** | 3,9 | 15,6 | ----- | 15,6 | ----- | ----- | ----- | ----- |
| **45 - 65** | 7,8 | 7,8 | ----- | 7,8 | ----- | ----- | ----- | ----- |
| **125** | 15,6 | 7,8 | ----- | 7,8 | ----- | ----- | ----- | ----- |
| | | | | | | | | |

| ***Staphylococcus aureus* ATTC 33592** | | | | | | | | |
|---|---|---|---|---|---|---|---|---|
| **-80** | 15,6 | 7,8 | ----- | 7,8 | ----- | ----- | ----- | ----- |
| **4 - 8** | 15,6 | 7,8 | ----- | 3,9 | ----- | ----- | ----- | ----- |
| **20 - 25** | 7,8 | 15,6 | ----- | 7,8 | ----- | ----- | ----- | ----- |
| **45 - 65** | 15,6 | 31,2 | ----- | 7,8 | ----- | ----- | ----- | ----- |
| **125** | 15,6 | 31,2 | ----- | 7,8 | ----- | ----- | ----- | ----- |
| | | | | | | | | |

| ***Staphylococcus hominis* AG 1115** | | | | | | | | |
|---|---|---|---|---|---|---|---|---|
| **-80** | ----- | ----- | 7,8 | 7,8 | 7,8 | 15,6 | 15,6 | 15,6 |
| **4 - 8** | ----- | ----- | 15,6 | 7,8 | 7,8 | 15,6 | 15,6 | 15,6 |
| **20 - 25** | ----- | ----- | 31,2 | 7,8 | 15,6 | 7,8 | 31,2 | 62,5 |
| **45 - 65** | ----- | ----- | 15,6 | 3,9 | 15,6 | 15,6 | 15,6 | 62,5 |
| **125** | ----- | ----- | 15,6 | 7,8 | 7,8 | 15,6 | 31,2 | 31,2 |
| | | | | | | | | |

| ***Escherichia coli* AG 1215** | | | | | | | | |
|---|---|---|---|---|---|---|---|---|
| **-80** | 62,5 | 125,0 | 62,5 | ----- | ----- | 15,6 | 62,5 | 62,5 |
| **4 - 8** | 62,5 | 62,5 | 62,5 | ----- | ----- | 62,5 | 15,6 | 62,5 |
| **20 - 25** | 250,0 | 125,0 | 31,2 | ----- | ----- | 31,2 | 31,2 | 125,0 |
| **45 - 65** | 62,5 | 250,0 | 31,2 | ----- | ----- | 62,5 | 62,5 | 125,0 |
| **125** | 125,0 | 125,0 | 62,5 | ----- | ----- | 31,2 | 62,5 | 125,0 |

### Beispiel 3 - Herstellung des Fosfomycin-Terbinafin-Kombinationspräparats

In 500 L Wasser für Injektionszwecke gemäß Ph. Eur. (8. Auflage) werden 26,4 kg Fosfomycin-Dinatrium (entspricht 20 kg Fosfomycin), 500 g Bernsteinsäure und 0,5 kg Terbinafin-HCl unter sterilen Bedingungen gelöst. Diese Lösung wird unter Sterilbedingungen in 50000 Flaschen (Einzelvolumen: 10 ml) abgefüllt. Die Flaschen werden mit je einem geeigneten Gummistopfen verschlossen und zu je 50 Stück in einen Karton verpackt.

Die in den Flaschen beinhaltete Lösung ist gebrauchsfertig zur inhalativen Verabreichung als Aerosol und kann monatelang bei Raumtemperatur gelagert werden.

## Patentansprüche

1. Geschlossener Behälter, beinhaltend eine wässrige Lösung gebrauchsfertig zur parenteralen Verabreichung, wobei in der Lösung zumindest ein pharmazeutisch annehmbares Salz von Fosfomycin und eine pharmazeutisch annehmbare Säure gelöst sind, wobei die Säure Bernsteinsäure ist, wobei die Lösung eine Fosfomycin-Gesamtdosis entsprechend 1 bis 15 Gramm Fosfomycin beinhaltet, wobei das Massenverhältnis von Bernsteinsäure zu Fosfomycin in der Lösung zwischen 30:1 und 50:1 liegt, wobei das Volumen der Lösung zwischen 1 ml und 100 ml / g Fosfomycin liegt.

2. Geschlossener Behälter gemäß Anspruch 1, wobei die Lösung gebrauchsfertig zur intravenösen Verabreichung vorliegt.

3. Geschlossener Behälter gemäß einem der Ansprüche 1 bis 2, wobei der geschlossene Behälter versiegelt ist,
vorzugsweise wobei der Behälter in einer Weise geschlossen ist, dass ein Öffnen des Behälters im Wesentlichen unwiderruflich ist.

4. Geschlossener Behälter gemäß einem der Ansprüche 1 bis 3, wobei der Behälter ausgewählt ist aus:
- einer Ampulle insbesondere aus Kunststoff oder Glas, vorzugsweise eine Brechampulle insbesondere aus Kunststoff oder Glas,
- einer Durchstechflasche, bevorzugt versiegelt mit einer Schutzkappe,
- einem Infusionsbeutel und
- einer Spritze, insbesondere einer Fertigspritze.

5. Geschlossener Behälter gemäß einem der Ansprüche 1 bis 4, wobei der Behälter, der vorzugsweise eine Durchstechflasche oder ein Infusionsbeutel ist, ein unversehrtes Septum beispielsweise aus Gummi aufweist.

6. Geschlossener Behälter gemäß einem der Ansprüche 1 bis 5, wobei der Behälter einen Lichtschutz für die Lösung darstellt, insbesondere im Wesentlichen lichtundurchlässig ist.

7. Geschlossener Behälter gemäß einem der Ansprüche 1 bis 6, wobei das pharmazeutisch annehmbare Salz von Fosfomycin ausgewählt ist aus Fosfomycin-Dinatriumsalz, Fosfomycin-Mononatriumsalz, einem Fosfomycin-Kaliumsalz, einem Fosfomycin-Lithiumsalz, Fosfomycin-Magnesiumsalz, und Fosfomycin-Calciumsalz.

8. Geschlossener Behälter gemäß einem der Ansprüche 1 bis 7, wobei die Lösung einen pH-Wert von 6,4 bis 8,4, bevorzugt von mehr als 6,5 und weniger als 8,0, eher bevorzugt von 6,9 bis 7,9, mehr bevorzugt von 7,0 bis 7,8, noch mehr bevorzugt von 7,1 bis 7,7, insbesondere von 7,2 bis 7,6 oder gar von 7,3 bis 7,5, aufweist; oder
wobei die Lösung einen pH-Wert von weniger als 10,0, bevorzugt von weniger als 9,0, mehr bevorzugt von weniger als 8,5, noch mehr bevorzugt von weniger als 8,0, insbesondere von weniger als 7,75 oder gar von höchstens 7,5 aufweist.

9. Geschlossener Behälter gemäß einem der Ansprüche 1 bis 8, wobei das Massenverhältnis von Bernsteinsäure zu Fosfomycin bei im Wesentlichen 40:1 liegt.

10. Geschlossener Behälter gemäß einem der Ansprüche 1 bis 9, wobei der geschlossene Behälter den am 15. Februar 2016 gültigen "EU Guidelines for Good Manufacturing Practice for Medicinal Products for Human and Veterinary Use", insbesondere Annex 1 in der Fassung "25 November 2008 (rev.)", entspricht; und/oder wobei der Behälter gemäß Annex 1 der "EU Guidelines for Good Manufacturing Practice for Medicinal Products for Human and Veterinary Use" in der Fassung "25 November 2008 (rev.)" mit der Lösung befüllt und geschlossen worden ist.

11. Verpackung, umfassend zumindest einen geschlossenen Behälter gemäß einem der Ansprüche 1 bis 10 und vorzugsweise eine Gebrauchsinformation;
wobei die Verpackung einen Lichtschutz für die Lösung bzw. den geschlossenen Behälter darstellt, insbesondere im Wesentlichen lichtundurchlässig ist, und/oder wobei der Behälter einen Lichtschutz für die Lösung darstellt, insbesondere im Wesentlichen lichtundurchlässig ist.

12. Verfahren, umfassend das Lagern des geschlossenen Behälters eines der Ansprüche 1 bis 10 bzw. der Verpackung von Anspruch 11 bei einer Temperatur zwischen 0°C und 60°C, insbesondere bei einer Temperatur von 15°C bis 35°C oder bei Raumtemperatur, für zumindest eine Woche, bevorzugt für zumindest einen Monat, mehr bevorzugt für zumindest drei Monate, wobei die Lagerung unter Lichtschutz statt findet.

13. Verfahren zur Herstellung des geschlossenen Behälters eines der Ansprüche 1 bis 10, umfassend das sterile Befüllen des Behälters mit der Lösung und das Schließen des Behälters, gegebenenfalls ferner umfassend das Versiegeln des geschlossenen Behälters;
vorzugsweise wobei die Lösung nicht aus einem steril entsprechend den am 15. Februar 2016 gültigen "EU Guidelines for Good Manufacturing Practice for Medicinal Products for Human and Veterinary Use", insbesondere entsprechend Annex 1 in der Fassung "25 November 2008 (rev.)", vermischten Pulvergemisch des pharmazeutisch annehmbaren Fosfomycin-Salzes und der pharmazeutisch annehmbaren Säure hergestellt wird.

## Claims

1. Closed container containing an aqueous solution ready for parenteral administration, wherein at least one pharmaceutically acceptable salt of fosfomycin and a pharmaceutically acceptable acid are dissolved in the solution, wherein the acid is succinic acid, wherein the solution contains a total dose of fosfomycin corresponding to 1 to 15 grams of fosfomycin, wherein the mass ratio of succinic acid to fosfomycin in the solution is between 30:1 and 50:1, wherein the volume of the solution is between 1 ml and 100 ml/g fosfomycin.

2. Closed container according to claim 1, wherein the solution is ready for intravenous administration.

3. Closed container according to either claim 1 or claim 2, wherein the closed container is sealed, preferably wherein the container is closed in such a manner that opening the container is substantially irreversible.

4. Closed container according to any of claims 1 to 3, wherein the container is selected from:
- an ampoule, in particular made of plastics material or glass, preferably a snap-off ampoule, in particular made of plastics material or glass,
- a puncturable vial, preferably sealed using a protective cap,
- an infusion bag and
- a syringe, in particular a prefilled syringe.

5. Closed container according to any of claims 1 to 4, wherein the container, which preferably is a puncturable vial or an infusion bag, has an intact septum, for example made of rubber.

6. Closed container according to any of claims 1 to 5, wherein the container provides light protection for the solution, and in particular is substantially opaque.

7. Closed container according to any of claims 1 to 6, wherein the pharmaceutically acceptable salt of fosfomycin is selected from fosfomycin disodium salt, fosfomycin monosodium salt, a fosfomycin potassium salt, a fosfomycin lithium salt, fosfomycin magnesium salt, and fosfomycin calcium salt.

8. Closed container according to any of claims 1 to 7, wherein the solution has a pH of 6.4 to 8.4, preferably of greater than 6.5 and less than 8.0, more preferably of 6.9 to 7.9, particularly preferably of 7.0 to 7.8 even more preferably of 7.1 to 7.7, in particular of 7.2 to 7.6 or even of 7.3 to 7.5;
or wherein the solution has a pH of less than 10.0, preferably of less than 9.0, more preferably of less than 8.5, even more preferably of less than 8.0, in particular of less than 7.75 or even of at most 7.5.

9. Closed container according to any of claims 1 to 8, wherein the mass ratio of succinic acid to fosfomycin is substantially 40:1.

10. Closed container according to any of claims 1 to 9, wherein the closed container complies with the "EU Guidelines for Good Manufacturing Practice for Medicinal Products for Human and Veterinary Use", valid on 15 February 2016, in particular Annex 1 in the version "25 November 2008 (rev.)";
and/or the wherein container has been filled with the solution and closed in accordance with Annex 1 of the "EU Guidelines for Good Manufacturing Practice for Medicinal Products for Human and Veterinary Use" in the version "25 November 2008 (rev.)".

11. Packaging comprising at least one closed container according to any of claims 1 to 10 and preferably a patient information leaflet;
wherein the packaging provides light protection for the solution and the closed container, in particular is substantially opaque, and/or wherein the container provides light protection for the solution, in particular is substantially opaque.

12. Method comprising storing the closed container of any of claims 1 to 10 or the packaging of claim 11 at a temperature of between 0°C and 60°C, in particular at a temperature of 15°C to 35°C or at room temperature, for at least one week, preferably for at least one month, particularly preferably for at least three months, wherein the storage takes place under light protection.

13. Method for producing the closed container of any of claims 1 to 10, comprising filling the container with the solution in a sterile manner, and closing the container, optionally further comprising sealing the closed container;
preferably wherein the solution is not produced from a powder mixture of the pharmaceutically acceptable fosfomycin salt and the pharmaceutically acceptable acid that are mixed in a sterile manner according to the "EU Guidelines for Good Manufacturing Practice for Medicinal Products for Human and Veterinary Use", valid on 15 February 2016, in particular according to Annex 1 in the version "25 November 2008 (rev.)" .

## Revendications

1. Récipient fermé, comprenant une solution aqueuse prête à l'emploi pour l'administration parentérale, au moins un sel pharmaceutiquement acceptable de fosfomycine et un acide pharmaceutiquement acceptable étant dissous dans la solution, l'acide étant l'acide succinique, la solution contenant une dose totale de fosfomycine correspondant à 1 à 15 grammes de fosfomycine, le rapport en poids de l'acide succinique sur la fosfomycine dans la solution étant compris entre 30:1 et 50:1, le volume de la solution étant compris entre 1 ml et 100 ml/g de fosfomycine.

2. Récipient fermé selon la revendication 1, dans lequel la solution se présente prête à l'emploi pour l'administration intraveineuse.

3. Récipient fermé selon l'une des revendications 1 à 2, ledit récipient fermé étant scellé, de préférence ledit récipient étant fermé de manière qu'une ouverture du récipient soit sensiblement irréversible.

4. Récipient fermé selon l'une des revendications 1 à 3, ledit récipient étant choisi parmi :
- une ampoule, notamment en plastique ou en verre, de préférence une ampoule à rompre, notamment en plastique ou en verre,
- un flacon pouvant être percé, de préférence scellé avec un capuchon protecteur,
- une poche de perfusion, et
- une seringue, notamment une seringue prête à l'emploi.

5. Récipient fermé selon l'une des revendications 1 à 4, ledit récipient, qui est de préférence un flacon pouvant être percé ou une poche de perfusion, présente un septum intact par exemple en caoutchouc.

6. Récipient fermé selon l'une des revendications 1 à 5, ledit récipient constituant une protection contre la lumière pour la solution, étant notamment sensiblement imperméable à la lumière.

7. Récipient fermé selon l'une des revendications 1 à 6, dans lequel le sel pharmaceutiquement acceptable de fosfomycine est choisi parmi le sel disodique de fosfomycine, le sel monosodique de fosfomycine, un sel potassique de fosfomycine, un sel de lithium de fosfomycine, un sel de magnésium de fosfomycine, et un sel de calcium de fosfomycine.

8. Récipient fermé selon l'une des revendications 1 à 7, dans lequel la solution présente un pH de 6,4 à 8,4, de préférence supérieur à 6,5 et inférieur à 8,0, de manière plutôt préférée de 6,9 à 7,9, de manière plus préférée de 7,0 à 7,8, de manière encore plus préférée de 7,1 à 7,7, notamment de 7,2 à 7,6 voire même de 7,3 à 7,5 ; ou
dans lequel la solution présente un pH inférieur à 10,0, de préférence inférieur à 9,0, de manière plus préférée inférieur à 8,5, de manière encore plus préférée inférieur à 8,0, notamment inférieure à 7,75 ou même d'au maximum 7,5.

9. Récipient fermé selon l'une des revendications 1 à 8, dans lequel le rapport en poids de l'acide succinique sur la fosfomycine est sensiblement de 40:1.

10. Récipient fermé selon l'une des revendications 1 à 9, ledit récipient fermé répondant aux « Directives de l'UE relatives aux bonnes pratiques de fabrication pour les médicaments à usage humain et vétérinaire » en vigueur au 15 février 2016, en particulier à l'Annexe 1 dans sa version du « 25 novembre 2008 (révisée) » ; et /ou
ledit récipient conforme à l'Annexe 1 des « Directives de l'UE relatives aux bonnes pratiques de fabrication pour les médicaments à usage humain et vétérinaire » dans sa version du « 25 novembre 2008 (révisée) » a été rempli avec la solution et a été fermé.

11. Conditionnement, comprenant au moins un récipient fermé selon l'une des revendications 1 à 10 et de préférence une notice d'utilisation ;
ledit conditionnement constituant une protection contre la lumière pour la solution et/ou le récipient fermé, étant notamment sensiblement imperméable à la lumière, et/ou le récipient constituant une protection contre la lumière pour la solution, étant notamment sensiblement imperméable à la lumière.

12. Procédé comprenant le stockage du récipient fermé selon l'une des revendications 1 à 10 et/ou du conditionnement selon la revendication 11 à une température comprise entre 0°C et 60°C, en particulier à une température de 15°C à 35°C ou à température ambiante, pendant au moins une semaine, de préférence pendant au moins un mois, de manière plus préférée pendant au moins trois mois, le stockage ayant lieu à l'abri de la lumière.

13. Procédé de fabrication du récipient fermé selon l'une des revendications 1 à 10, comprenant le chargement du récipient sous environnement stérile avec la solution et la fermeture du récipient, le cas échéant en outre comprenant le scellement du récipient fermé ;
de préférence dans lequel la solution n'est pas fabriquée à partir d'un mélange pulvérulent du sel pharmaceutiquement acceptable de fosfomycine et de l'acide pharmaceutiquement acceptable mélangé sous environnement stérile conformément aux « Directives de l'UE relatives aux bonnes pratiques de fabrication pour les médicaments à usage humain et vétérinaire » en vigueur au 15 février 2016, en particulier à l'Annexe 1 dans sa version du « 25 novembre 2008 (révisée) ».
